# FASCICULE DE BREVET EUROPEEN

(11) **EP 3 027 123 B1**
(45) Date de publication et mention de la délivrance du brevet: **13.02.2019**
(21) Numéro de dépôt: 14748244.2
(22) Date de dépôt: 09.07.2014
(51) Int. Cl.: A61B 17/11

(54) **DISPOSITIF ANASTOMOTIQUE POUR JOINDRE DES LUMENS OU DES VISCERES ENTRE-EUX**
ANASTOMIEVORRICHTUNG ZUM VERBINDEN VON LUMINA ODER EINGEWEIDEN MITEINANDER
ANASTOMOTIC DEVICE FOR JOINING LUMENS OR VISCERA TO EACH OTHER

(30) Priorité: 01.08.2013 FR 1357621
(43) Date de publication de la demande: 08.06.2016
(73) Titulaire: Cousin Biotech, 59117 Wervicq Sud (FR); Université de Lille, 59800 Lille (FR)
(72) Inventeur: DALLE, Valery, 59170 Croix (FR); SOLECKI, Gilles, 59390 Lannoy (FR); PATTOU, François, 59000 Lille (FR); HIMPENS, Jacques, 9921 Vinderhoute (BE); CAIAZZO, Robert, 59800 Lille (FR)
(74) Mandataire: Balesta, Pierre
(86) Numéro de dépôt international: PCT/FR2014/051755
(87) Numéro de publication internationale: WO 2015/015080

(56) Documents cités:
- DE-A1- 10 325 128
- FR-A1- 2 976 782
- US-A- 5 941 908
- US-A1- 2002 082 614
- US-A1- 2011 264 186
- US-B1- 6 527 800

## Description

### Arrière-plan de l'invention

La présente invention concerne le domaine technique des dispositifs anastomotiques pour joindre des lumens ou des viscères creux, aptes à être déployés à la surface de lumens adjacents superposés.

L'anastomose chirurgicale est un abouchement chirurgical de deux conduits, réalisé par suture manuelle ou par agrafage. La suture est effectuée avec du fil qualifié de résorbable, car il disparait de lui-même avec le temps à l'intérieur de l'organisme. Ces sutures sont utilisées en chirurgie digestive (relative à un acte de chirurgie sur l'estomac, le duodénum, l'intestin grêle, le colon ou l'anus), en chirurgie bariatrique, mais également en chirurgie urologique en ce qui concerne les voies urinaires. Quelquefois, les anastomoses chirurgicales se font avec du fil non résorbable, en particulier s'agissant de l'anastomose des vaisseaux, notamment des artères.

L'anastomose dans le domaine de la chirurgie digestive est utilisée pour rétablir la continuité du tube digestif. Le tube digestif, composé du duodénum, de l'intestin grêle, et du colon, peut être obturé par une néoplasie. Il est donc nécessaire de rétablir le flux alimentaire en réséquant la partie du tube digestif obturée et de réaliser ensuite un abouchement des deux extrémités libres du tube digestif. Cet abouchement est réalisé à l'aide d'une agrafeuse circulaire ou manuellement par sutures.

L'anastomose dans le domaine de la chirurgie bariatrique a pour objectif de traiter chirurgicalement l'obésité. Une des techniques chirurgicales est la diversion pancréatique ou opération de Scopinaro. Cette technique chirurgicale consiste en particulier à réaliser une poche en enlevant une grande partie de l'estomac et en la raccordant, par une anastomose, à la dernière partie de l'intestin grêle, mettant ainsi la majeure partie de l'intestin hors circuit. Les aliments consommés ne sont donc que très faiblement digérés.

Les différents types d'anastomoses digestives sont les suivantes :
- l'anastomose oeso-gastrique se fait entre l'oesophage et l'estomac,
- l'anastomose gastro-jéjunale se fait entre l'estomac et le jéjunum,
- l'anastomose duodeno-jéjunale se fait entre le duodénum et le jéjunum,
- l'anastomose iléocolite se fait entre l'iléon et le côlon,
- l'anastomose colo-colique se fait entre deux parties du côlon,
- l'anastomose iléo-rectale se fait entre l'iléon et le rectum.

Les dispositifs implantables permettant de réaliser des anastomoses gastro-intestinales par voie endoscopique trans-oesophagienne permettent dans un premier temps une nécrose ischémique des tissus par compression, laquelle partie nécrosée tombe ensuite dans l'intestin grêle après maturation de la nécrose. La maturation de la nécrose peut être définie comme l'instant à partir duquel les tissus nécrosés peuvent être séparés facilement des tissus vascularisés.

Les documents EP 1.790.297 A1 et WO 03/000142 ont pour objet des dispositifs anastomotiques comprenant un tube constitué de fils à mémoire de forme entrecroisés et formés au vu de la structure illustrée pour WO 03/000142 à la figure 2 et EP 1.790.297 A1 à la figure 8, soit par tressage, soit manuellement en faisant s'entrecroiser des fils sur un support comportant des clous.

Ces dispositifs de forme annulaire, tel que cela est visible par exemple à la figure 5C pour EP 1.790.297 A1 ou à la figure 3B s'agissant de WO 03/000142, présentent en fonctionnement des pétales s'entrecroisant et se projetant selon leur périphérie externe.

Le document US 2002/0082614 divulgue un dispositif anastomotique ayant un corps tubulaire formé à partir d'un membre tubulaire dans lequel une découpe laser a été effectuée. Le dispositif comprend des jeux de projections radiales, proximales et distales s'étendant dudit corps tubulaire selon au moins une profondeur (E) respectivement en périphérie desdites première et seconde extrémités, les jeux distal et proximal étant séparés par une distance interne minimum (D), et comprenant aussi un jeu de projections radiales intermédiaire s'étendant dudit corps selon au plus une profondeur (e) et disposé entre les jeux de projections radiales, proximales et distales, avec la profondeur (e) inférieure à (E), et avec la distance interne minimum (D) déterminée en sorte de recevoir deux lumens adjacents superposés.

Les tissus gastriques et intestinaux placés entre ces pétales se nécrosent par compression, puis la partie nécrosée tombe avec le dispositif dans le système digestif pour laisser en place une ouverture appelée communément une anastomose.

Juste après leur implantation et avant cicatrisation des tissus, ces dispositifs peuvent glisser de leur site d'implantation, de sorte que la liaison physique établie entre l'estomac et l'intestin grêle n'est alors plus assurée. Les sécrétions gastriques et intestinales s'écoulent alors dans la cavité péritonéale. Le patient peut alors mourir d'une péritonite ou d'une infection généralisée (septicémie).

Après plusieurs mois d'implantation et suite à la nécrose des tissus superposés et placés entre ces pétales, ces dispositifs sont conçus pour tomber dans l'intestin grêle. Ils sont, de ce fait, potentiellement dangereux pour le patient. En effet, ces dispositifs une fois tombés dans l'intestin grêle, peuvent se bloquer dans le système digestif engendrant une occlusion du conduit intestinal et provoquant ensuite une perforation intestinale.

En outre, la corrosion des alliages titane/nickel utilisés dans ces dispositifs par l'acidité gastrique provoque des casses de fils à de multiples endroits après une durée d'implantation supérieure à 120 jours. L'extrémité agressive de ces fils cassés peut perforer les organes ou se planter dans la paroi intestinale. Lorsque ces dispositifs tombent dans l'intestin, les extrémités des fils peuvent se planter dans la paroi de ce dernier et se fixer définitivement créant alors une occlusion intestinale. Les conséquences de cette occlusion peuvent être mortelles.

En outre, l'anastomose réalisée avec cette technique ne permet pas de créer une ouverture pérenne entre l'estomac et l'intestin grêle. L'ouverture anastomotique créée se referme car les tissus que se forment sont d'origine fibrotique.

Dans les dispositifs décrits dans les documents EP 1.790.297 et WO 03/000142, l'effort de compression dépend de l'épaisseur des parois gastrique et intestinale maintenues ensemble, puisque l'effort compressif assuré par les pétales à la périphérie externe desdits dispositifs est homogène.

La maturation de la nécrose se fera ainsi rapidement si l'épaisseur de ces parois est importante. Par contre, cette maturation de la nécrose arrivera plus tardivement si l'épaisseur de ces parois est plus faible.

Ce constat implique que ce type de dispositif anastomotique n'est pas conçu pour être retiré de façon fiable d'un patient à l'autre. En effet, si ce dispositif est placé sur un patient dont les épaisseurs des parois gastrique et intestinale sont faibles et que le gastro-entérologue souhaite retirer l'implant à 90 jours, il existe un risque important que lors de l'intervention chirurgicale, consistant à ôter le dispositif anastomotique afin de libérer l'anastomose créée, la nécrose ne soit pas mature et que le chirurgien ne parvienne pas à retirer ledit dispositif. Si ce même dispositif est placé sur un patient dont les épaisseurs des parois gastrique et intestinale réunies sont importantes, le gastro-entérologue souhaitant retirer l'implant également à 90 jours, il existe alors un risque que la nécrose soit déjà mâture et que le dispositif soit tombé dans le système digestif.

Ces dispositifs anastomotiques ont également pour inconvénient que leur allongement est faible lorsqu'ils passent de leur position au repos, donc déployée, à leur position comprimée dans un cathéter d'introduction. Cet allongement mesuré, tel que cela est décrit ci-après dans le présent texte, sur le dispositif anastomotique comparativement entre ses positions au repos et disposé en force dans un cathéter d'introduction, est compris entre 20 % et 70 %. En outre, ces dispositifs, dans leur position comprimée dans ledit cathéter d'introduction, ne mesurent que 2 ou 3 cm, ce qui ne donne que très peu de marge de manoeuvre au gastro-entérologue lorsque le dispositif sort du cathéter d'introduction pour être placé au niveau des parois anatomiques à rapprocher.

Ces dispositifs ne permettent pas ainsi une disposition sur le site d'implantation précise, fiable et reproductible.

Le dispositif anastomotique selon la présente invention permet de contrôler la nécrose des parois anatomiques réunis en sorte que le chirurgien puisse retirer l'implant à 90 jours d'implantation et que cette nécrose soit mature.

En outre, le dispositif anastomotique selon la présente invention permet de sécuriser immédiatement après implantation la liaison gastro-intestinale. Ledit dispositif sera ensuite retiré après une durée d'implantation déterminée, qui peut être de l'ordre de 90 jours, pour permettre aux muqueuses intestinales de reprendre leur place et laisser en place une anastomose pérenne.

Le document WO 2012/175847 A1 divulgue un dispositif anastomotique comprenant un corps tubulaire principal tricoté et dont les extrémités supérieure et inférieure sont recourbées sur elles-mêmes en sorte de former deux surfaces d'appui venant pincer entre-elles les parois anatomiques à rapprocher.

Ce type de dispositif ne permet pas un contrôle différencié précis de la compression exercée sur les parois anatomiques à rapprocher et à nécroser de sorte que tout comme les dispositifs précédents de l'état de l'art, la nécrose n'est pas correctement maitrisée. Il existe ainsi un risque que ce dispositif tombe dans le système digestif en engendrant les problèmes ci-dessus développés.

### Objet et résumé de l'invention

La présente invention a pour objet un dispositif anastomotique pour joindre des lumens ou des viscères creux, aptes à être déployés à la surface de deux lumens adjacents superposés, en particulier pour l'anastomose digestive, notamment entre l'estomac et l'intestin grêle, ledit dispositif implantable comprend un corps tubulaire principal d'axe longitudinale L, ayant des première et seconde extrémités ouvertes ainsi que des faces intérieure et extérieure opposées.

Ledit corps tubulaire principal comprend au moins trois colonnes de boucles et au moins trois rangées de boucles formées à partir d'un ou plusieurs monofilaments et/ou plusieurs fils multifilamentaires.

Avantageusement, le corps tubulaire comprend des jeux de projections radiales, proximales et distales s'étendant dudit corps tubulaire selon au moins une profondeur E, respectivement en périphérie desdites première et seconde extrémités, lesdits jeux distal et proximal étant séparés par une distance interne minimum D.

Ledit dispositif comprend un jeu de projections radiales intermédiaire s'étendant dudit corps selon au plus une profondeur e et disposée entre les jeux de projections radiales, proximales et distales, la profondeur e est inférieure à la profondeur E, en outre la distance interne minimum D est déterminée en sorte de recevoir des lumens adjacents superposés, en particulier la distance interne minimum D est supérieure ou égale à la somme des épaisseurs des lumens adjacents superposés.

Les jeux proximal, distal et intermédiaire comprennent des projections radiales respectivement proximales, distales et intermédiaires.

Les profondeurs e et E sont définies comme étant les distances séparant le point le plus éloigné d'une projection radiale par rapport au centre du corps tubulaire, par exemple dans le cas d'un pétale le sommet de ce dernier, et le plan passant par deux colonnes de mailles adjacentes entre lesquelles s'étend ladite projection radiale, la distance étant mesurée selon une direction perpendiculaire à ce plan et passant par ce dit point.

La distance interne D est la distance minimum séparant une projection radiale distale d'une projection radiale proximale.

Les jeux de projections radiales, proximales et distales n'ont pas pour fonction d'exercer un effet compressif mais permettent de sécuriser immédiatement la liaison entre les deux lumens adjacents superposés, tels que la paroi gastrique et la paroi intestinale, après implantation du dispositif anastomotique. La distance interne D entre les projections radiales des jeux distal et proximal est sensiblement égale ou supérieure à l'épaisseur des parois gastrique et intestinale réunies afin d'éviter leur compression. Avantageusement, cette disposition permet également de maintenir le dispositif anastomotique en liaison avec les parois de l'estomac et de l'intestin grêle jusqu'au retrait du dispositif, par exemple après 90 jours ou à 120 jours ou plus d'implantation.

Les projections radiales du jeu intermédiaire assurent la compression des tissus permettant ainsi leur nécrose. Les projections radiales intermédiaires ne pincent pas les parois anatomiques à rapprocher entre-elles mais exercent une compression dans leur épaisseur.

Avantageusement, la profondeur e des projections radiales intermédiaires inférieure à la profondeur E des projections radiales, distales et proximales combinée à leur disposition entre les projections radiales, distales et proximales, favorisent une nécrose ischémique des tissus orientée dans une direction sensiblement perpendiculaire à l'axe longitudinal L, également désignée dans la suite du présent texte par nécrose radiale, et ce sur une profondeur e inférieure à E. Cette disposition facilite le retrait du dispositif anastomotique à plus de 90 jours, et notamment à plus de 120 jours, et évite en outre que ce dernier ne tombe dans l'intestin grêle.

En outre, la succession de projections radiales intermédiaires ne s'entrecroisant pas permet d'assurer une force de compression constante et indépendante de l'épaisseur des parois gastriques et intestinales. Avantageusement, cette disposition permet de maitriser l'apparition de la nécrose ainsi que sa durée de maturation.

De préférence, le dispositif anastomotique selon l'invention est destiné à être implanté et retiré par endoscopie trans-oesophagienne sans ouverture par laparotomie.

La première durée de 90 jours correspond à la durée de maturation de la nécrose qui peut être définie comme la durée de compression minimale nécessaire pour nécroser par ischémie les tissus des parois gastrique et intestinale et permettre ensuite de séparer facilement ces tissus nécrosés de la partie vascularisée saine.

L'effort pour séparer facilement le dispositif anastomotique selon l'invention du site de l'anastomose par endoscopie gastro-oesophagienne est de préférence inférieur à 10 Newtons.

Entre 90 jours et 120 jours d'implantation, le dispositif anastomotique selon l'invention ne pourra pas tomber dans l'intestin grêle malgré la maturation de la nécrose car il est retenu par les projections radiales, proximales et distales, qui ne nécrosent pas les tissus, ou très faiblement.

De préférence, l'endoscopie trans-oesophagienne met en oeuvre un endoscope double-canal de type Olympus GIF 2T160 qui possède un canal opérateur de diamètre interne 2,8 mm et un autre canal opérateur de diamètre interne 3,7 mm.

Le corps tubulaire principal selon l'invention est réalisé de préférence par la technique de bonneterie tubulaire trame ou autrement dit par la technique de tricotage tubulaire en mailles cueillies.

Il est également possible de former le corps tubulaire principal manuellement en formant des colonnes et des rangées de boucles avec un fil, par exemple à l'aide d'un support clouté.

On entend au sens de la présente invention par boucles, toutes boucles de mailles formant une unité structurelle d'un segment de fils comprenant une tête, deux ailes ou jambes, et deux pieds, la tête étant la partie supérieure de la maille formant un demi-cercle, les deux ailes ou jambes forment la partie étroite de la maille reliant la tête et les pieds et les deux pieds étant les parties inférieures incurvées de la maille.

De préférence, lesdites boucles de mailles sont des mailles cueillies, en particulier la partie inférieure incurvée ou les pieds de la boucle de maille est intégrée à la moitié d'une boucle d'entre-mailles.

De préférence, le corps tubulaire principal comprend des colonnes et rangées de boucles formées à l'aide d'un ou plusieurs monofilaments et/ou plusieurs fils multifilamentaires, de préférence un ou plusieurs fils monofilaments, notamment dans un alliage super élastique, tel qu'un alliage comprenant du titane et du nickel, de préférence à proportions égales en poids.

Un alliage super-élastique de titane et de nickel est en partie caractérisé par sa température de fin de transition austénite, communément appelée Af. Elle correspond à la température de fin de changement de phase entre la phase martensite et la phase austénite. Dans sa phase martensite, l'alliage de titane et de nickel est malléable et dans sa phase austénite, l'alliage est très élastique. Dans la plage de températures dans laquelle la phase austénite est stable, c'est-à-dire pour une température supérieure ou égale à Af, la transformation martensitique peut être aussi provoquée sous l'action d'une contrainte. La superplasticité se caractérise alors par une récupération immédiate et complète du fil lorsque la contrainte appliquée cesse. La contrainte s'exerce dans ce cas dans le domaine de déformation élastique de l'austénite. La température Af caractérisant la transition du fil superélastique de la présente invention est comprise entre - 10°C et 5°C et permet d'avoir le comportement super-élastique recherché. Un fil à mémoire de forme peut être défini par une température Af supérieur à 5°C. Ce fil dit à mémoire de forme ne convient pas dans le cadre de la présente invention car l'effort de compression radiale du dispositif anastomotique n'est pas suffisant.

De préférence, le ou lesdits fils monofilaments ont un diamètre compris dans l'intervalle [0.15 mm ; 0.35 mm], encore de préférence dans l'intervalle [0.2 mm ; 0.25 mm].

De préférence, le ou lesdits fils multifilamentaires ont un titre compris dans l'intervalle [114 tex, 620 tex], pour un nombre de filaments par fil compris dans l'intervalle [2 ; 10].

Dans une variante de réalisation, les projections radiales sont formées par des segments reliant les colonnes de boucles, lesdites colonnes de boucles ayant un axe longitudinal ℓ sensiblement parallèle à l'axe longitudinal L du corps tubulaire.

Les segments reliant les colonnes de boucles sont de préférence formés dans un fil monofilamentaire ou multifilamentaire, et encore de préférence dans un fil monofilamentaire, tel que dans un alliage super élastique, notamment en titane-nickel, de préférence en proportions égales en poids, et encore de préférence sensiblement 50 % de titane pour 50% de nickel en poids par rapport au poids total du fil.

Dans une variante de réalisation, chaque jeu de projections radiales, qu'il soit distal, proximal ou intermédiaire, comprend au moins deux rangées de boucles, chaque rangée comprenant des projections radiales, lesdites projections radiales étant disposées dans des plans (p), lesdits plans (p) étant séquents avec l'axe longitudinal L.

Dans une sous-variante, les projections radiales des jeux proximal et distal sont disposés dans des plans (p) sensiblement parallèles entre-eux et perpendiculaires à l'axe longitudinal L.

Dans une sous-variante, les projections radiales du jeu intermédiaire sont dans des plans (p) sensiblement perpendiculaires à l'axe longitudinale L lorsque le dispositif est au repos, c'est-à-dire dans une position déployé, et dans des plans (p) formant un angle β compris dans l'intervalle [10° ; 85°] avec l'axe longitudinale L en fonctionnement, c'est-à-dire lorsqu'une compression radiale est exercée sur lesdites projections intermédiaires. Dans une variante de réalisation, les projections radiales de deux rangées de boucles adjacentes sont distantes d'une distance d correspondant sensiblement à la hauteur d'une boucle.

Ces différentes dispositions permettent de mieux contrôler la compression exercée par chacune des projections puisque ces dernières ne s'entrecroisent pas contrairement aux dispositifs anastomotiques dans l'état de la technique.

Dans une variante de réalisation, les projections radiales du jeu distal et/ou proximal, éventuellement les projections radiales du jeu intermédiaire, ont une forme de pétale, notamment une forme choisie, indépendamment d'une projection à l'autre, parmi les formes suivantes : un arc de cercle, une forme semi-circulaire, une forme semi-elliptique et une forme parabolique.

La configuration particulière du corps tubulaire principal selon l'invention et la disposition des rangées et colonnes de boucles permettent une multiplicité de la forme des projections radiales, puisque ces dernières sont indépendantes les unes des autres.

Dans une variante de réalisation, la profondeur E est égale ou supérieure à deux fois la profondeur e, de préférence supérieure ou égale à trois fois, encore de préférence supérieure ou égale à cinq fois, et encore de préférence supérieure ou égale à huit fois la profondeur e.

Dans une variante de réalisation, le corps tubulaire principal présente un allongement longitudinal selon l'axe L supérieur ou égal à 200 % à +/- 20 % près, de préférence supérieur ou égal à 380 % à +/- 20 % près.

Cette disposition permet au dispositif anastomotique selon l'invention de s'allonger de façon importante pour passer dans un cathéter de diamètre interne au moins égal à 3,0 mm.

Le pourcentage d'allongement axial du dispositif anastomotique entre sa position déployée, c'est à dire au repos avant son implantation, et sa position comprimée dans un cathéter de diamètre interne de 3,0 mm est compris entre 380 % et 520 % à +/- 20 % près.

Cet allongement est mesuré en comparant l'allongement au repos du dispositif anastomotique et celui obtenu suite à son placement en force dans un cathéter d'introduction de diamètre interne de 3,0 mm pour un diamètre externe de 3,50mm, notamment dans un polymère de polyéther bloc amide (PEBA), le conduit intérieur étant revêtu d'un polymère fluoré, en particulier d'un film de polytétrafluoroéthylène (PTFE) ayant une épaisseur d'environ 50 µm.

Cet allongement permet une grande précision de placement du dispositif selon l'invention par endoscopie trans-oesophagienne.

Cet allongement important est obtenu par la combinaison des rangées de boucles et des colonnes de boucles, de préférence formé par tricotage, en particulier par la formation de mailles cueillies, avec l'emploi d'un fil dans un alliage super élastique de titane-nickel.

Cette disposition permet une grande reproductivité de la technique opératoire.

Dans une variante de réalisation, le corps tubulaire principal comprend entre cinq colonnes de boucles et dix colonnes de boucles, et entre trois et six projections radiales par rangées de boucles.

Ainsi en fonction du nombre de colonnes de boucles, il est possible d'ajuster le nombre de projections radiales par rangées de boucles, le nombre de projections radiales étant inférieur ou égal au nombre de colonnes de boucles.

En effet, selon le procédé de fabrication retenu pour le corps tubulaire principal, il est possible de prévoir un nombre de projections radiales par rangées de boucles inférieur au nombre de segments disponibles sur une rangée de boucles, à savoir donc inférieur au nombre de colonnes de boucles disponibles.

Les inventeurs ont déterminé que cet agencement permet un compromis entre la force de compression suffisante pour créer une nécrose ischémique, la formation d'une liaison immédiate avec les parois de l'estomac et de l'intestin grêle ainsi qu'un encombrement minimum permettant d'optimiser l'allongement du dispositif anastomotique dans le cathéter d'introduction.

Dans une variante de réalisation, le corps tubulaire principal comprend de une à dix projections radiales disposées entre deux colonnes de boucles adjacentes, lesdites projections appartenant au jeu distal et proximal et/ou intermédiaire.

Dans une variante de réalisation, les jeux de projections radiales, distales et proximales comprennent chacun au moins deux rangées de boucles, et lorsque le jeu de projections radiales intermédiaires comprend de trois à huit rangées de boucles, lesdites rangées comprenant de trois à huit projections radiales.

Dans une variante, la distance minimum D est comprise dans l'intervalle [4 mm ; 20 mm], de préférence dans l'intervalle [6 mm ; 16 mm]. Dans une variante de réalisation, le corps tubulaire principal a un diamètre intérieur a compris dans l'intervalle [10 mm ; 30 mm], de préférence dans l'intervalle [20 mm ; 25 mm]

Dans une variante de réalisation, les colonnes et les rangées de boucles du corps tubulaire principal sont formées à partir d'un ou plusieurs fils monofilaments et/ou un ou plusieurs fils multifilamentaires, dans un alliage de titane/nickel super élastique.

### Brève description des dessins

- La figure **1** est une représentation schématique et vue de dessus d'un premier exemple de dispositif anastomotique de l'art antérieur ;
- La figure **2** est une représentation schématique et en perspective vue de côté d'un second exemple de dispositif anastomotique de l'art antérieur ;
- La figure **3** représente de façon schématique le comportement d'un dispositif anastomotique de l'art antérieur, tel que celui illustré à la figure **1****,** vue de côté, sur le site de l'anastomose, lors du maintien en compression des parois de l'estomac et de l'intestin grêle ; pour le cas où les épaisseurs des parois gastrique et viscérale réunies sont importantes.
- La figure **4A** est une représentation schématique et en perspective d'un premier exemple d'un dispositif anastomotique selon l'invention ;
- La figure **4B** est une représentation schématique et vue de dessus du dispositif anastomotique représenté à la figure **4A** ;
- La figure **5** est une représentation schématique du dispositif anastomotique représenté aux figures **4A** et **4B** sur le site de l'anastomose ;
- La figure **6** représente en perspective de façon schématique le placement du dispositif anastomotique représenté aux figures **4A****,** **4B et 5** sur le site d'implantation par voie trans-oesophagienne à l'aide d'un endoscope mettant en oeuvre un cathéter d'introduction de diamètre interne de 3,0 mm ;
- La figure **7** est une représentation schématique et en perspective d'un second exemple de réalisation d'un dispositif anastomotique selon l'invention ;
- La figure **8** est une représentation schématique et vue du dessus du dispositif anastomotique représenté à la figure **7****.**

### Description détaillée de l'invention

Le premier exemple de dispositif anastomotique **1** de l'art antérieur comprend une périphérie interne **la** et une périphérie externe **1b** ainsi que des pétales inférieures **2b** et supérieures **2a.**

Ce dispositif **1** est adapté pour nécroser les tissus. Lorsque les tissus se nécrosent dans le temps, le dispositif **1** peut être séparé des tissus sains sur le site de l'anastomose et peut ainsi passer à travers le système digestif du patient.

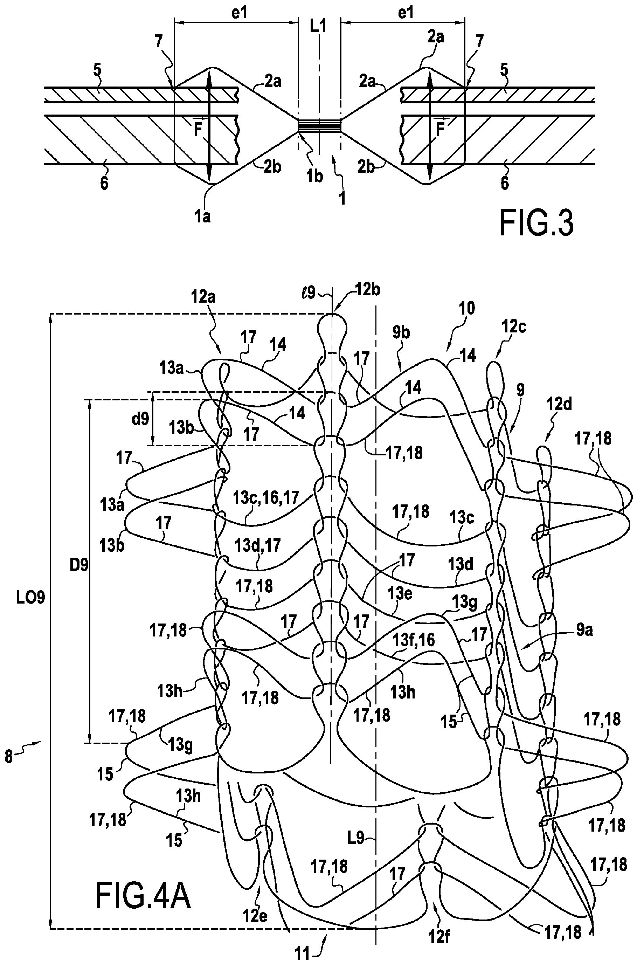

Ce dispositif **1** correspond à la figure **3A** du document EP 1.790.297 A1.

La périphérie interne **la** correspond à la portion du tube à partir duquel le dispositif **1** a été formé replié sur lui-même et les extrémités supérieure et inférieure du tube ouvert rapprochées correspondent à la périphérie externe **1b.**

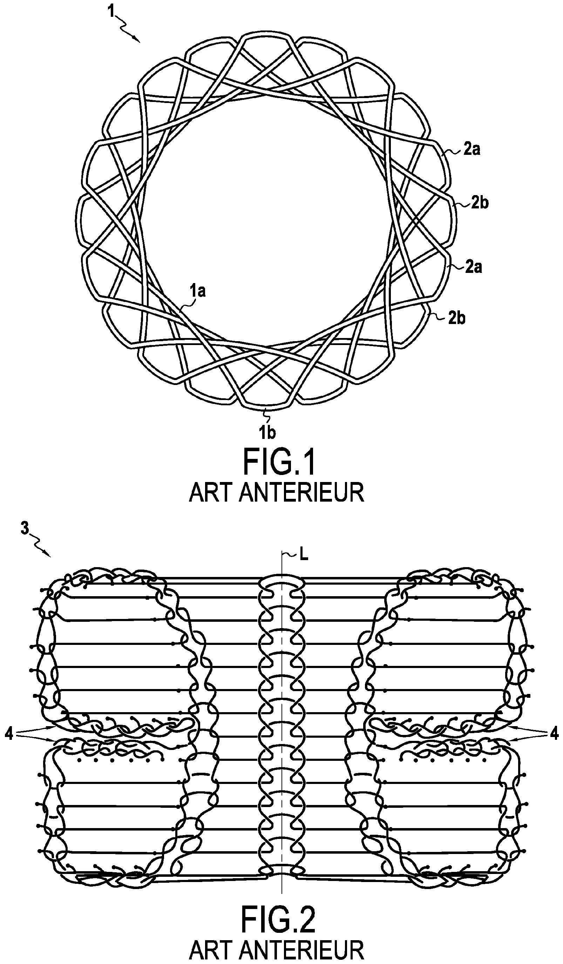

Le dispositif anastomotique **3** représenté à la figure **2** est un second exemple de dispositif de l'art antérieur et comprend des faces d'appui **4** orientées l'une au regard de l'autre, lesquelles vont venir pincer les parois de l'estomac et de l'intestin grêle entre elles.

La figure **3** représente le dispositif anastomotique **1** en fonctionnement, c'est-à-dire dans une position dans laquelle il est déployé et maintient ensembles les parois anatomiques **5 et 6** correspondant respectivement à la paroi intestinale et à la paroi gastrique.

On remarque que la profondeur des pétales supérieure **2a** et inférieure **2b** est la même et correspond sensiblement à l'épaisseur **e1** mesurée à partir de l'axe longitudinal **L1** du dispositif **1,**

La périphérie externe **la** du dispositif **1** va ainsi nécroser les parties superposées des parois **5** et **6** au niveau des zones annulaires **7.**

Une fois la nécrose terminée, le dispositif **1** tombera dans le système digestif du patient occasionnant les risques ci-dessus évoqués.

Le dispositif décrit à la figure **2** se comportera de la même façon avec les faces d'appui **4** l'une au regard de l'autre.

Le premier exemple de dispositif anastomotique **8** selon l'invention représenté à la figure **4A** pour joindre des lumens ou des viscères creux, dans cet exemple précis les parois de l'estomac **20** et de l'intestin grêle **19,** comprend un corps tubulaire principal **9** d'axe longitudinal **L₉** ayant des première **10** et seconde **11** extrémités ouvertes ainsi que des faces extérieure **9a** et intérieure **9b** opposées. Ledit corps tubulaire principal **9** comprend, dans cet exemple précis, six colonnes de boucles **12a-12f** et huit rangées de boucles **13a-13h.**

Le corps tubulaire principal **9** comprend des jeux de projections radiales, proximales **14** et distales **15** s'étendant dudit corps tubulaire **9** selon une profondeur **E₉** respectivement en périphérie desdites première **10** et seconde **11** extrémités ouvertes. Lesdits jeux distal **15** et proximal **14** sont séparés par une distance interne minimum **D₉.**

Le corps tubulaire principal **9** comprend un jeu de projections radiales intermédiaires **16** s'étendant dudit corps tubulaire **9** selon une profondeur **e₉** et disposée entre les jeux de projections radiales, proximales **14** et distales **15.** La profondeur **e₉** est inférieure à **E₉.**

En outre, la distance interne minimum **D₉** est déterminée en sorte de recevoir des lumens adjacents superposés, à savoir les parois anatomiques à rapprocher en l'occurrence la paroi intestinale **19** et la paroi gastrique **20, D₉** est déterminée en sorte d'être supérieure ou égale à la somme des épaisseurs des parois intestinale **19** et gastrique **20.**

Les projections radiales **17** sont formées par des segments **18** reliant les colonnes de boucles **12a** à **12f,** lesquelles colonnes de boucles **12a** à **12f** ont un axe longitudinal **ℓ₉** sensiblement parallèles à l'axe longitudinal **L₉** du corps tubulaire **9.**

Les jeux de projections radiales **17,** proximal **14** et distal **15** comprennent chacun, dans cet exemple précis, deux rangées de boucles, à savoir respectivement les rangées de boucles **13a** et **13b,** et **13g** et **13h,** lesdites projections radiales **17** de chaque rangée **13a, 13b, 13g, 13h** sont disposées dans des plans **p1, p2** et **p7, p8** sensiblement parallèles, lesdits plans **p1, p2, p7, p8** étant séquents avec l'axe longitudinal **L₉,** en particulier au repos, comme en fonctionnement, sensiblement perpendiculaires à l'axe longitudinale **L₉.**

Le jeu de projections radiales intermédiaires **16** comprend, dans cet exemple précis, quatre rangées de boucles **13c, 13d, 13e, 13f** chaque rangée **13c-13f** comprend des projections radiales **17,** lesquelles projections radiales **17** de chaque rangée **13c-13f** sont disposées respectivement dans des plans **p3, p4, p5, p6,** lesdits plans **p3, p4, p5, p6** forment un angle **β9** compris dans l'intervalle [10° ; 85°] avec l'axe longitudinal **L₉.**

Les projections radiales **17** de deux rangées de boucles adjacentes, par exemple **13a** et **13b,** sont distantes d'une distance **d₉** correspond sensiblement à la hauteur d'une boucle.

Dans cet exemple précis, les projections radiales **17** des jeux distal **15,** proximal **14** et intermédiaire **16** ont une forme de pétale.

La profondeur **E₉** est supérieure ou égale à quatre fois la profondeur **e₉,** dans cet exemple précis **E₉** est égale à cinq fois la profondeur **e₉.**

Le corps tubulaire principal **9** comprend huit projections radiales disposées entre deux colonnes de boucles adjacentes, telles que par exemples les colonnes **12a** et **12b,** lesdites projections **17** appartenant aux jeux proximal **14,** distal **15** et intermédiaire **16.**

Dans cet exemple précis, le diamètre interne **a₉** est de l'ordre de 25 mm, la distance minimum **D9** entre les premières projections radiales **17** des jeux proximal **14** et distal **15** est de l'ordre de 10 mm, enfin la longueur **L₉** au repos du corps tubulaire **9** du dispositif **8** est de l'ordre de 20 mm.

Le pourcentage d'allongement axial du dispositif **8** est de l'ordre de 400 % +/- 20 % près.

Cet allongement est mesuré sur le dispositif anastomotique **8** et effectué par placement du dispositif **8** dans un cathéter de faible diamètre interne, de l'ordre de 3,0 mm, pour un diamètre externe de 3,50 mm.

Ce cathéter est en polyéther bloc-amide (PEBA) et présente une surface interne revêtue d'un polymère fluoré en particulier de polytétra-fluoréthylène tel que le téflon ayant une épaisseur de l'ordre de 50µm.

L'introduction en force de la totalité du dispositif anastomotique **8** dans un tel cathéter engendre son allongement, lequel correspond à son allongement à l'état comprimé **Lc9.**

On obtient ainsi une différence d'allongement entre l'allongement au repos du dispositif anastomotique **L₉,** de l'ordre de 2 cm, à une longueur dans un état comprimé **Lc₉** de l'ordre de 10 cm, correspondant à la latitude de placement disponible **La9,** de l'ordre de 8 cm, pour le chirurgien lorsque le dispositif anastomotique 8 sort de l'introducteur sur le site d'implantation.

La longueur à l'état comprimé du dispositif **8** est mesurée à l'aide d'une règle graduée à travers l'épaisseur de la paroi du cathéter d'introduction.

Pour comparaison, le dispositif anastomotique **1** représenté à la figure **1** a une longueur **L1** au repos de l'ordre de 2 cm et une longueur **LC1** à l'état comprimé dans ledit cathéter d'introduction de l'ordre de 3,4 cm ce qui représente une latitude de placement de l'ordre de 1,4 cm et un allongement de 70 %.

On comprend bien ainsi que le dispositif anastomotique **8** selon l'invention offre au praticien davantage de liberté et de fiabilité dans son placement au niveau des parois anatomiques **19** et **20** à maintenir et nécroser. Le très faible allongement des dispositifs anastomotiques de l'état de la technique engendrent un risque que ce dernier, lorsqu'il se déploie en sortie de l'introducteur sur le site d'implantation, soit libéré trop vite puisqu'il ne peut être maintenu que sur une très faible longueur et que, dès lors, il tombe directement dans le système digestif du patient.

Le diamètre interne du dispositif **1** est également de l'ordre de 25 mm.

Il s'agit ici du diamètre interne du tube à partir duquel le dispositif **1** est mis en forme, et en particulier à la périphérie interne **la** du dispositif **1.**

Le corps tubulaire **9** est, dans cet exemple précis, de préférence fabriqué avec une technique de bonneterie tubulaire trame ou par une technique de tricotage tubulaire en mailles cueillies avec un fil monofilamentaire de diamètre externe 0,25 mm, de préférence dans un alliage à mémoire de forme à base de titane et de nickel, et encore de préférence dans un alliage super-élastique.

Tel que cela est visible à la figure **4A****,** chaque boucle présente une tête, des jambes ou ailes et des pieds de mailles.

Les boucles des colonnes et des rangées sont communes et se retrouvent à l'intersection des colonnes **12a-12f** et des rangées **13a-13h.**

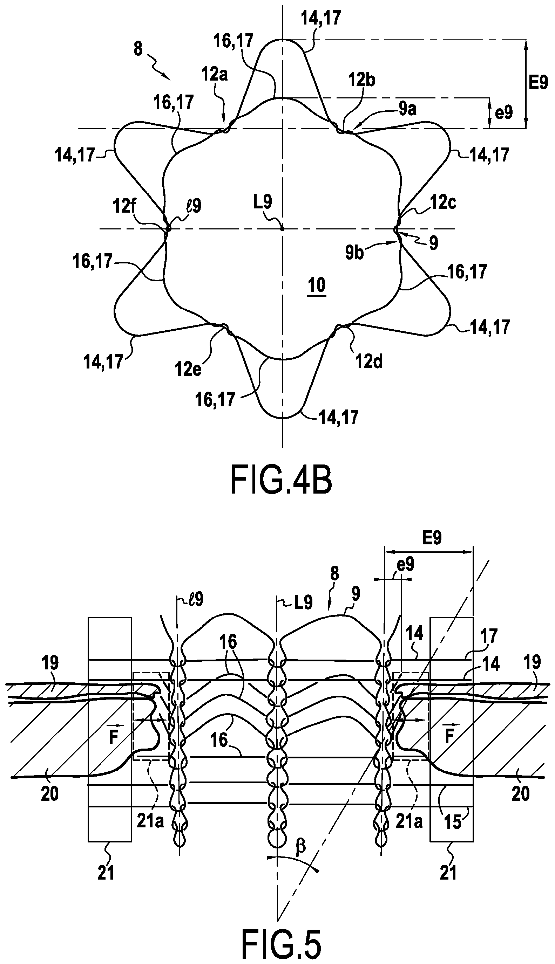

La figure **4B** représente vue de dessus le dispositif anastomotique **8,** la profondeur **e₉** correspond à la profondeur des projections radiales **17** du jeu intermédiaire **16.**

La figure **5** représente le dispositif anastomotique 8 sur le site d'implantation dans une position dans laquelle il maintient la paroi intestinale **19** et la paroi gastrique **20** ensembles.

On observe ainsi que les projections radiales **17** des jeux proximal **14** et distal **15** maintiennent ensemble, sans exercer d'effet compressif, les parois intestinale **19** et gastrique **20.** Les projections radiales **17** du jeu intermédiaire **16** exercent quant à elles un effet compressif au regard des parois anatomiques **19** et **20.**

L'effet compressif est exercé selon les flèches **f** pour chacune des projections radiales **17,** l'effet compressif est ici exercé selon une direction **f** qui est sensiblement perpendiculaire à l'axe **L9** du corps tubulaire **9** de sorte que les parois intestinale **19** et gastrique **20** ne sont pas nécrosées sur toute la zone **21** en particulier selon toute l'épaisseur des tissus **19, 20** dans la direction axiale (correspondant à **L9**). Les tissus **19, 20** seront nécrosés en particulier selon les zones **21a** correspondant à la profondeur **e9** des projections radiales **17** intermédiaires **16.**

Les jeux de projections radiales **17,** distales **15** et proximales **14** permettent de maintenir les parois anatomiques **19** et **20** ensembles sans exercer d'effet compressif afin de sécuriser cette liaison anatomique dans les premiers instants après une implantation par voie trans-oesophagienne tandis que les projections radiales **17** du jeu intermédiaire **16** exercent un effet compressif orienté principalement selon les flèches **f** dans une direction sensiblement perpendiculaire à l'axe longitudinal **L9** du dispositif anastomotique **9,** ce qui permet de créer une nécrose préférentiellement radiale et ainsi facilite le retrait du dispositif au bout de 90 jours, ou encore à 120 jours.

Avantageusement, les jeux de projections radiales **17,** distales **15** et proximales **14** permettent également de maintenir le dispositif anastomotique sur les parois anatomiques **19** et **20,** quand bien même la nécrose parvient à maturité.

La nécrose obtenue avec le dispositif anastomotique **8** est davantage une nécrose radiale contrairement aux nécroses axiales obtenues dans l'état de la technique, ce qui facilite le retrait du dispositif à 90 jours voire jusqu'à 120 jours et évite que ce dernier ne tombe dans le système digestif du patient et notamment se détache des parois anatomiques **19** et **20** laissant ainsi le chirurgien procéder à ce retrait.

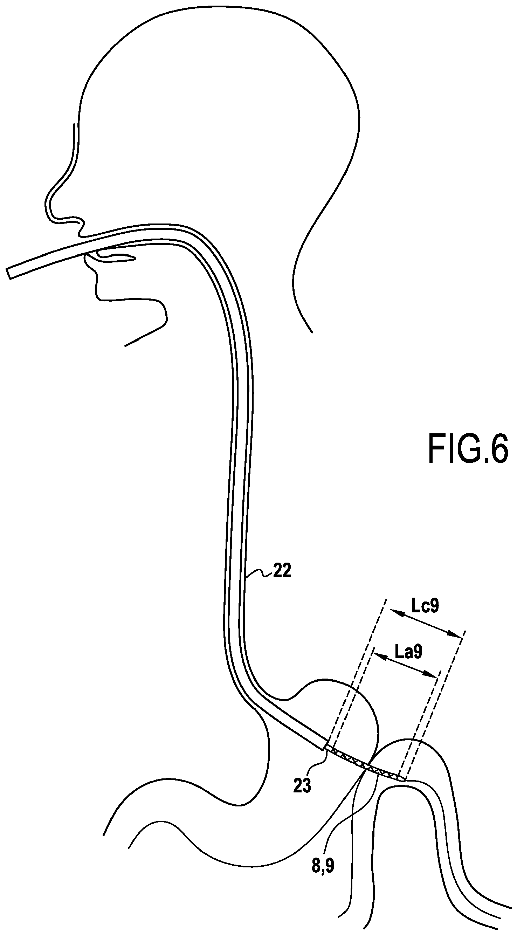

La figure **6** représente de manière schématique la disposition sur le site anastomotique du dispositif **8.** On visualise ainsi que lors de l'extraction du dispositif **9** de l'endoscope **22** et en particulier du cathéter **23,** le praticien dispose d'une latitude de placement **Lag** de l'ordre de 8 cm, ce qui lui permet de correctement positionner les projections radiales des jeux distal **14** et proximal **15** sans que ledit dispositif **9** ne risque de tomber dans le système digestif du patient.

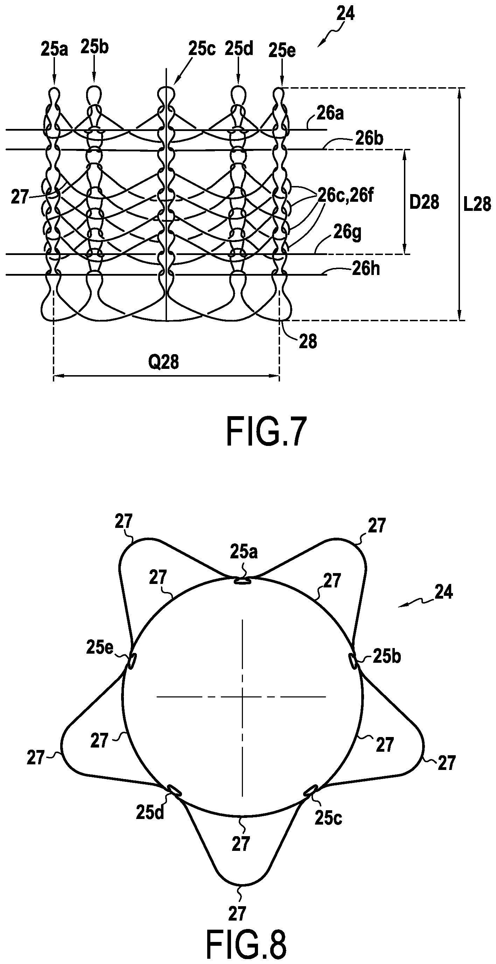

Le second exemple de dispositif anastomotique **24** selon la présente invention est représenté aux figures **7** et **8** et comprend **5** colonnes de boucles **25a, 25b, 25c, 25d, 25e** ainsi que huit rangées de boucles **26a-26h.**

Le dispositif anastomotique **24** diffère du dispositif anastomotique **9** de par le nombre de colonnes de boucles.

La disposition des projections radiales **27** est ici la même que les projections radiales **17.**

La diminution du nombre de colonnes de boucles permet de diminuer l'encombrement du dispositif anastomotique **24** pour un diamètre interne **a28** du corps tubulaire **28** de l'ordre de 25 mm, soit du même ordre que **a9** et permet d'obtenir une latitude de placement **La28** plus importante, à savoir de l'ordre de 10 cm.

En effet, l'allongement obtenu pour le dispositif anastomotique **24** est ici de l'ordre de 500 %, +/- 20 %, mesuré de la même manière que ci-dessus décrit à l'aide d'un cathéter d'introduction.

La distance minimale **D28** est ici de l'ordre de 7 mm, la longueur **L28** du corps tubulaire **28** est de l'ordre de 20 mm et la longueur **LC28** à l'état comprimé du dispositif **24** est de l'ordre de 120 mm.

## Revendications

1. Dispositif anastomotique pour joindre des lumens ou des viscères creux, apte à être déployé à la surface de deux lumens adjacents superposés, ledit dispositif comprend un corps tubulaire principal d'axe longitudinal (L), ayant des première et seconde extrémités ouvertes ainsi que des faces extérieure et intérieure opposées, ledit corps tubulaire principal comprenant au moins trois colonnes de boucles et au moins trois rangées de boucles formées à partir d'un ou plusieurs monofilaments et/ou plusieurs fils multifilamentaires, **caractérisé en ce que** ledit corps tubulaire comprend des jeux de projections radiales, proximales et distales s'étendant dudit corps tubulaire selon au moins une profondeur (E) respectivement en périphérie desdites première et seconde extrémités, lesdits jeux distal et proximal étant séparés par une distance interne minimum (D), **en ce qu'**il comprend un jeu de projections radiales intermédiaire s'étendant dudit corps selon au plus une profondeur (e) et disposé entre les jeux de projections radiales, proximales et distales, **en ce que** (e) est inférieure à (E), et **en ce que** la distance interne minimum (D) est déterminée en sorte de recevoir deux lumens adjacents superposés.

2. Dispositif anastomotique selon la revendication 1, **caractérisé en ce que** les projections radiales sont formées par des segments reliant les colonnes de boucles, lesquelles colonnes de boucles ont un axe longitudinal (I) sensiblement parallèle à l'axe longitudinal (L) du corps tubulaire.

3. Dispositif anastomotique selon l'une ou l'autre des revendications 1 et 2, **caractérisé en ce que** chaque jeu de projections radiales comprend au moins deux rangées de boucles, chaque rangées comprenant des projections radiales, lesdites projections radiales étant disposées dans des plans (p), lesdits plans (p) étant sécants avec l'axe longitudinal (L).

4. Dispositif anastomotique selon l'une quelconque des revendications 1 à 3, **caractérisé en ce que** les projections radiales des jeux proximal et distal sont disposés dans des plans (p) sensiblement parallèles entre-eux et perpendiculaires à l'axe longitudinal L.

5. Dispositif anastomotique selon l'une quelconque des revendications 1 à 4, **caractérisé en ce que** les projections radiales du jeu intermédiaire sont dans des plans (p) sensiblement perpendiculaires à l'axe longitudinale L lorsque le dispositif est au repos, c'est-à-dire dans une position déployé, et dans des plans (p) formant un angle β compris dans l'intervalle [10° ; 85°] avec l'axe longitudinale L en fonctionnement, c'est-à-dire lorsqu'une compression radiale est exercée sur lesdites projections intermédiaires.

6. Dispositif anastomotique selon l'une quelconque des revendications 1 à 5, **caractérisé en ce que** les projections radiales de deux rangées de boucles adjacentes sont distantes d'une distance (d) correspondant sensiblement à la hauteur d'une boucle.

7. Dispositif anastomotique selon l'une quelconque des revendications 1 à 6, **caractérisé en ce que** les projections radiales des jeux distal et/ou proximal, éventuellement les projections radiales du jeu intermédiaire, ont une forme de pétales, notamment une forme choisie, indépendant d'une projection à l'autre, parmi les formes suivantes ; un arc de cercle, une forme semi-circulaire, une forme semi-elliptique et une forme parabolique.

8. Dispositif anastomotique selon l'une quelconque des revendications précédentes **caractérisé en ce que** la profondeur (E) est supérieure ou égale à deux fois la profondeur (e), de préférence supérieure ou égale à huit fois la profondeur (e).

9. Dispositif anastomotique selon l'une quelconque des revendications 1 à 8, **caractérisé en ce que** le corps tubulaire principal présente un allongement longitudinal selon l'axe (L) supérieure ou égale à 200 % à +/- 20 % près, de préférence supérieure ou égale à 380 % à +/- 20 % près.

10. Dispositif anastomotique selon l'une quelconque des revendications 1 à 9, **caractérisé en ce que** le corps tubulaire comprend entre cinq colonnes de boucles et dix colonnes de boucles, et entre trois et six projections radiales par rangée de boucles.

11. Dispositif anastomotique selon l'une quelconque des revendications 1 à 10, **caractérisé en ce que** le corps tubulaire principal comprend de une à dix projections radiales disposées entre deux colonnes de boucles adjacentes, lesdites projections appartenant au(x) jeu(x) distal et/ou proximal et/ou intermédiaire.

12. Dispositif anastomotique selon l'une quelconque des revendications 1 à 11, **caractérisé en ce que** les jeux de projections radiales, distales et proximales comprennent chacun au moins deux rangées de boucles, et **en ce que** le jeu de projections radiales intermédiaire comprend de trois à huit rangées de boucles, lesdites rangées comprenant de trois à huit projections radiales.

13. Dispositif anastomotique, selon l'une quelconque des revendications 1 à 12, **caractérisé en ce que** la distance minimum (D) est comprise dans l'intervalle [4 mm ; 20 mm].

14. Dispositif anastomotique, selon l'une quelconque des revendications 1 à 13, **caractérisé en ce que** le corps tubulaire principal a un diamètre intérieur (a) compris dans l'intervalle [10 mm ; 25 mm].

15. Dispositif anastomotique selon l'une quelconque des revendications précédentes, **caractérisé en ce que** les colonnes et les rangées de boucles sont formées dans un alliage de titane-nickel super-élastique.

## Patentansprüche

1. Anastomievorrichtung zum Verbinden von Lumina oder hohlen Eingeweiden, die geeignet ist, auf der Oberfläche von zwei übereinander liegenden benachbarten Lumina entfaltet zu werden, wobei die Vorrichtung einen röhrenförmigen Hauptkörper der Längsachse (L) aufweist, der ein erstes und zweites offenes Ende sowie eine äußere und innere Fläche, die gegenüberliegend sind, aufweist, wobei der röhrenförmige Hauptkörper mindestens drei Säulen von Schlaufen und mindestens drei Reihen von Schlaufen aufweist, die aus einem oder mehreren Monofilamenten und/oder mehreren Multifilamentgarnen gebildet sind, **dadurch gekennzeichnet, dass** der röhrenförmige Körper Sätze von radialen, proximalen und distalen Vorsprüngen aufweist, die sich von dem röhrenförmigen Körper in mindestens einer Tiefe (E) jeweils an der Peripherie des ersten und des zweiten Endes erstrecken, wobei der distale und der proximale Satz durch einen minimalen inneren Abstand (D) getrennt sind, dadurch, dass er einen Zwischensatz von radialen Vorsprüngen aufweist, die sich von dem Körper in höchstens einer Tiefe (e) erstrecken, und zwischen den Sätzen von radialen, proximalen und distalen Vorsprüngen angeordnet ist, dadurch dass (e) kleiner als (E) ist, und dadurch, dass der minimale innere Abstand (D) derart bestimmt ist, um zwei übereinander liegende benachbarte Lumina aufzunehmen.

2. Anastomievorrichtung gemäß Anspruch 1, **dadurch gekennzeichnet, dass** die radialen Vorsprünge durch Segmente gebildet sind, die die Säulen von Schlaufen verbinden, wobei die Säulen von Schlaufen eine Längsachse (I) aufweisen, die im Wesentlichen parallel zu der Längsachse (L) des röhrenförmigen Körpers ist.

3. Anastomievorrichtung gemäß einem der Ansprüche 1 und 2, **dadurch gekennzeichnet, dass** jeder Satz von radialen Vorsprüngen mindestens zwei Reihen von Schlaufen aufweist, wobei jede Reihe radiale Vorsprünge aufweist, wobei die radialen Vorsprünge in Ebenen (p) angeordnet sind, wobei sich die Ebenen (p) mit der Längsachse (L) schneiden.

4. Anastomievorrichtung gemäß einem der Ansprüche 1 bis 3, **dadurch gekennzeichnet, dass** die radialen Vorsprünge des distalen und des proximalen Satzes in Ebenen (p) angeordnet sind, die im Wesentlichen untereinander parallel und senkrecht zu der Längsachse (L) sind.

5. Anastomievorrichtung gemäß einem der Ansprüche 1 bis 4, **dadurch gekennzeichnet, dass** die radialen Vorsprünge des Zwischensatzes in Ebenen (p), die im Wesentlichen senkrecht zu der Längsachse L sind, wenn die Vorrichtung in Ruhe ist, das heißt in einer entfalteten Position, und in Ebenen (p) sind, die einen Winkel β, der im Bereich [10°; 85°] liegt, mit der Längsachse L im Betrieb bilden, das heißt wenn eine radiale Kompression auf die Zwischenvorsprünge ausgeübt wird.

6. Anastomievorrichtung gemäß einem der Ansprüche 1 bis 5, **dadurch gekennzeichnet, dass** die radialen Vorsprünge von zwei benachbarten Reihen von Schlaufen um einen Abstand (d) beabstandet sind, der im Wesentlichen der Höhe einer Schlaufe entspricht.

7. Anastomievorrichtung gemäß einem der Ansprüche 1 bis 6, **dadurch gekennzeichnet, dass** die radialen Vorsprünge des distalen und/oder proximalen Satzes, eventuell die radialen Vorsprünge des Zwischensatzes, eine Form von Blütenblättern aufweisen, insbesondere eine Form, die unabhängig von einem Vorsprung zum anderen aus den folgenden Formen ausgewählt ist: einem Kreisbogen, einer halbkreisförmigen Form, einer semi-elliptischen Form und einer parabolischen Form.

8. Anastomievorrichtung gemäß einem der vorhergehenden Ansprüche, **dadurch gekennzeichnet, dass** die Tiefe (E) größer oder gleich dem Zweifachen der Tiefe (e), vorzugsweise größer oder gleich dem Achtfachen der Tiefe (e) ist.

9. Anastomievorrichtung gemäß einem der Ansprüche 1 bis 8, **dadurch gekennzeichnet, dass** der röhrenförmige Hauptkörper eine Längsdehnung entlang der Achse (L) aufweist, die größer oder gleich 200 % bis etwa +/- 20 %, vorzugsweise größer oder gleich 380 % bis etwa +/- 20 %, ist.

10. Anastomievorrichtung gemäß einem der Ansprüche 1 bis 9, **dadurch gekennzeichnet, dass** der röhrenförmige Körper zwischen fünf Säulen von Schlaufen und zehn Säulen von Schlaufen und zwischen drei und sechs radialen Vorsprüngen pro Reihe von Schlaufen aufweist.

11. Anastomievorrichtung gemäß einem der Ansprüche 1 bis 10, **dadurch gekennzeichnet, dass** der röhrenförmige Hauptkörper von einem bis zehn radiale Vorsprünge aufweist, die zwischen zwei benachbarten Säulen von Schlaufen angeordnet sind, wobei die Vorsprünge zu dem (den) distalen Satz (Sätzen) und/oder dem proximalen Satz und/oder Zwischensatz gehören.

12. Anastomievorrichtung gemäß einem der Ansprüche 1 bis 11, **dadurch gekennzeichnet, dass** die Sätze von radialen, distalen und proximalen Vorsprüngen jeweils mindestens zwei Reihen von Schlaufen aufweisen und dadurch, dass der Zwischensatz von radialen Vorsprüngen von drei bis acht Reihen von Schlaufen aufweist, wobei die Reihen von drei bis acht radiale Vorsprünge aufweisen.

13. Anastomievorrichtung gemäß einem der Ansprüche 1 bis 12, **dadurch gekennzeichnet, dass** der Mindestabstand (D) im Bereich [4 mm; 20 mm] liegt.

14. Anastomievorrichtung gemäß einem der Ansprüche 1 bis 13, **dadurch gekennzeichnet, dass** der röhrenförmige Hauptkörper einen Innendurchmesser (a) aufweist, der im Bereich [10 mm; 25 mm] liegt.

15. Anastomievorrichtung gemäß einem der vorhergehenden Ansprüche, **dadurch gekennzeichnet, dass** die Säulen und die Reihen von Schlaufen aus einer superelastischen Titan-Nickel-Legierung gebildet sind.

## Claims

1. An anastomosis device for joining together hollow lumens or viscera, the device being suitable for being deployed at the surface of two superposed adjacent lumens, said device comprising a main tubular body of longitudinal axis (L), having first and second open ends and opposite outside and inside faces, said main tubular body comprising at least three columns of loops and at least three rows of loops formed from one or more monofilament and/or one or more multifilament yarns, **characterized in that** said tubular body further comprises sets of proximal and distal radial projections extending from said tubular body along at least a depth (E) at the peripheries of said first and second ends respectively, said distal and proximal sets being spaced apart by a minimum internal distance (D), **in that** it comprises a set of intermediate radial projections extending from said body along at most a depth (e) and arranged between the sets of proximal and distal radial projections, **in that** (e) is less than (E), and **in that** the minimum internal distance (D) is determined so as to receive two superposed adjacent lumens.

2. An anastomosis device according to claim 1, **characterized in that** the radial projections are formed by segments that connect the columns of loops, which columns of loops have a longitudinal axis (ℓ) that is substantially parallel to the longitudinal axis (L) of the tubular body.

3. An anastomosis device according to either claim 1 or claim 2, **characterized in that** each set of radial projections comprises at least two rows of loops, each row comprising radial projections, said radial projections being arranged in planes (p), said planes (p) intersecting the longitudinal axis (L).

4. An anastomosis device according to any one of claims 1 to 3, **characterized in that** the radial projections of the proximal and distal sets are arranged in planes (p) that are substantially parallel to one another and perpendicular to the longitudinal axis L.

5. An anastomosis device according to any one of claims 1 to 4, **characterized in that** the radial projections of the intermediate set are in planes (p) that are substantially perpendicular to the longitudinal axis L when the device is at rest, i.e. in a deployed position, and in planes (p) that form an angle β, lying in the range [10° ; 85°], with the longitudinal axis L in operation, i.e. when radial compression is exerted on said intermediate projections.

6. An anastomosis device according to any one of claims 1 to 5, **characterized in that** the radial projections of two adjacent rows of loops are spaced apart by a distance (d) that corresponds substantially to the height of a loop.

7. An anastomosis device according to any one of claims 1 to 6, **characterized in that** the radial projections of the distal and/or proximal set(s), and possibly the radial projections of the intermediate set, have a shape of petals, in particular a shape that is selected, independently from one projection to another, from among the following shapes: a circular arc, a semi-circular shape, a semi-elliptical shape, and a parabolic shape.

8. An anastomosis device according to any preceding claim, **characterized in that** the depth (E) is greater than or equal to twice the depth (e), preferably greater than or equal to eight times the depth (e).

9. An anastomosis device according to any one of claims 1 to 8, **characterized in that** the main tubular body presents longitudinal elongation along axis (L) that is greater than or equal to 200% to within ± 20%, preferably greater than or equal to 380% to within ± 20%.

10. An anastomosis device according to any one of claims 1 to 9, **characterized in that** the tubular body comprises five to ten columns of loops, and three to six radial projections per row of loops.

11. An anastomosis device according to any one of claims 1 to 10, **characterized in that** the main tubular body includes one to ten radial projections arranged between two adjacent columns of loops, said projections belonging to the distal and/or proximal and/or intermediate set(s).

12. An anastomosis device according to any one of claims 1 to 11, **characterized in that** each of the sets of distal and proximal radial projections comprises at least two rows of loops, and **in that** the set of intermediate radial projections comprises three to eight rows of loops, said rows comprising three to eight radial projections.

13. An anastomosis device according to any one of claims 1 to 12, **characterized in that** the minimum distance (D) lies in the range [4 mm ; 20 mm].

14. An anastomosis device according to any one of claims 1 to 13, **characterized in that** the main tubular body has an inside diameter (a) lying in the range [10 mm ; 25 mm].

15. An anastomosis device according to any preceding claim, **characterized in that** the columns and the rows of loops are formed in a super-elastic titanium-nickel alloy.
